# EUROPEAN PATENT APPLICATION

(11) **EP 4 799 552 A1**
(43) Date of publication of application: **02.09.2026**
(21) Application number: 25160879.0
(22) Date of filing: 28.02.2025
(51) Int. Cl.: A61B 3/10

(54) **OPHTHALMIC IMAGING DEVICE**

(71) Applicant: Optos plc, Dunfermline, Scotland KY11 8GR (GB)
(72) Inventor: MIZUTA, Masahiro, Konan Minato-ku, Tokyo 108-6290 (JP)
(74) Representative: Hoffmann Eitle

(57) **Abstract**

An ophthalmic imaging device comprising: an imaging device that is arranged to project light from a light source onto an examined eye, and that is further arranged to detect light from the examined eye to acquire an ophthalmic image of the examined eye, wherein: the imaging device comprises: a scanner arranged to scan light from the light source, and a wide-angle optical system that projects light scanned by the scan section onto a retina of the examined eye at a wide angle; the wide-angle optical system comprising: a first elliptical mirror arranged to reflect light scanned by the scanner, and a second elliptical mirror arranged to reflect light from the first elliptical mirror so as to reflect light toward the examined eye, wherein: the second elliptical mirror comprises an elliptical profile in a first plane extending through a first focal point and a second focal point, and a circular profile in a second plane that is orthogonal to the first plane; and a first line extending between the first focal point and the second focal point is a substantially equal length to a second line extending perpendicularly from the first line to an apex of the second elliptical mirror.

## Description

### FIELD

The present disclosure generally relates to an ophthalmic imaging device. In particular, but not exclusively, it relates to a mirror-based ophthalmic imaging device.

### BACKGROUND

Ophthalmic imaging devices, such as scanning laser ophthalmoscopes (SLO), are used by clinicians to capture ophthalmic images of a patient's eye. For example, an ophthalmic imaging device may be used to acquire a fundus image of a patient's retina. A clinician may then review the fundus image to assess the ocular health of the patient.

Ophthalmic imaging devices may be wide-field or ultra-wide field of view imaging devices that can acquire wide-field or ultra-wide field fundus images. The Optos Daytona (RTM) is an example of an ultra-wide field ophthalmic imaging device that is arranged to acquire ultra-wide field fundus images of a patient's retina. Such ophthalmic imaging devices generally comprise a light source for outputting an imaging beam for imaging the patient's retina. The imaging beam is conveyed to and from the patient's eye by an optical system comprising a combination of optical elements and scan elements. In wide-field or ultra-wide field of view ophthalmic imaging devices the optical system that conveys the imaging beam to and from the patient's eye may comprise one or more concave or ellipsoidal mirrors. An example of an ultra-wide field mirror based ophthalmic imaging system is described in US 7959290.

Mirror-based ophthalmic imaging systems beneficially allow clinicians to capture ultra-wide field of view fundus images with a field of view in the region of 200°. However, the curvature of the mirrors used in mirror-based ophthalmic imaging systems can introduce aberrations to the imaging beam which can negatively impact the quality of ophthalmic images captured by the mirror-based ophthalmic imaging system. The invention aims to suppress or remove aberrations in the ophthalmic images captured by the ophthalmic imaging device by the geometrical architecture of a mirror-based imaging system.

### SUMMARY

A first aspect is an ophthalmic device including an imaging device that projects light from a light source onto an examined eye, and that also photo-detects light from the examined eye and captures an image of the examined eye. The imaging device includes a scan section that scans light from the light source, and a wide-angle optical system that projects light scanned by the scan section onto the examined eye at a wide angle. The wide-angle optical system includes a first elliptical mirror that reflects light scanned by the scan section, and a second elliptical mirror that reflects light from the first elliptical mirror so as to reflect light toward the examined eye. For a first light that is on a first side looking from center light of a scanning center by the scan section, d1 is a distance from a first condensing spot to a first reflection point on the first elliptical mirror, d2 is a distance from the first reflection point to a second condensing spot, d3 is a distance from the second condensing spot to a second reflection point of the second elliptical mirror, and d4 is a distance from the second reflection point to a third condensing spot. For a second light that is on a second side, which is the opposite side to the first side, looking from the center light of the scanning center by the scan section, d1' is a distance from a first condensing spot to a first reflection point on the first elliptical mirror, d2' is a distance from the first reflection point A to a second condensing spot, d3' is a distance from the second condensing spot to a second reflection point of the second elliptical mirror, and d4' is a distance from the second reflection point to a third condensing spot. The first elliptical mirror and the second elliptical mirror are set such that either of the following sets of relationships holds: d1, d2, d3, and d4 are related by d2 being shorter than d1 and d3 being shorter than d4, and d1', d2', d3', and d4' are related by d1' being shorter than d2' and d4' being shorter than d3'; or d1, d2, d3, and d4 are related by d2 being longer than d1 and d3 being longer than d4, and d1', d2', d3', and d4' are related by d1' being longer than d2' and d4' being longer than d3'.

A second aspect is an ophthalmic device including an imaging device that projects light from a light source onto an examined eye, and that also photo-detects light from the examined eye and captures an image of the examined eye. The imaging device includes a scan section that scans light from the light source, and a wide-angle optical system that projects light scanned by the scan section onto the examined eye at a wide angle. The wide-angle optical system includes a first elliptical mirror that reflects light scanned by the scan section, and a second elliptical mirror that reflects light from the first elliptical mirror so as to reflect light toward the examined eye. The second elliptical mirror includes an elliptical profile in a first cross-section taken through a first focal point and a second focal point, and a true circle profile in a second cross-section orthogonal to the first cross-section, and a length of a perpendicular line dropped to an apex of the second elliptical mirror from a line segment connecting the first focal point and the second focal point is substantially the same as a radius of the true circle.

### BRIEF DESCRIPTION OF THE DRAWINGS

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic diagram of an ophthalmic imaging system according to a first exemplary embodiment.
Figure 2 is a schematic diagram of an ophthalmic imaging device according to the first exemplary embodiment.
Figure 3 is a schematic diagram of a server.
Figure 4 is an explanatory diagram of functions implemented by an image processing program in a CPU of a server.
Figure 5 is a schematic diagram of an imaging optical system comprising a wide-angle optical system.
Figure 6 is a schematic diagram of a wide-angle optical system of the first exemplary embodiment.
Figure 7 is a schematic diagram of a wide-angle optical system of the first exemplary embodiment.
Figure 8 is a schematic diagram of a wide-angle optical system of a second exemplary embodiment.
Figure 9 is a schematic configuration diagram to explain a configuration of a wide-angle optical system of the second exemplary embodiment.

### DETAILED DESCRIPTION

Detailed explanation follows regarding exemplary embodiments to implement technology disclosed herein, with reference to the drawings. Note that the same reference numerals are appended throughout the drawings to features, elements and processing modules with the same operation, and performing the same function, and duplicate explanation thereof will be omitted as appropriate. Moreover, sometimes description will be omitted for features not directly related to the technology disclosed herein and for known features. The dimensions and proportions in the drawings may be exaggerated for ease of explanation, and sometimes differ from actual proportions. Furthermore, each of the drawings are merely illustrated schematically to such a degree as to enable sufficient understanding of the technology disclosed herein. The technology disclosed herein is accordingly not limited to the examples illustrated.

The technology disclosed herein is applicable to any ophthalmic imaging device according to the technology disclosed herein. The ophthalmic imaging device may be an imaging device for acquiring images related to eyes, such as fundus images, optical coherence tomography (OCT) images or the like. In order to simplify the following explanation, the present exemplary embodiments will be described in the context of: an ophthalmic imaging device arranged to acquire ophthalmic images, and/or to an ophthalmic imaging device for an observer such as a clinician to observe an eye of a patient or the like (hereafter referred to as an examined eye). For example, the clinician may observe the examined eye to view the fundus of the eye, the periphery of the retina, or an anterior eye portion. The clinician may observe the eye for the purpose of assessing the ocular health of the patient, for the purpose of eye diagnostics in ophthalmology and/or for the purpose of surgical procedures to the eye.

### First Exemplary Embodiment

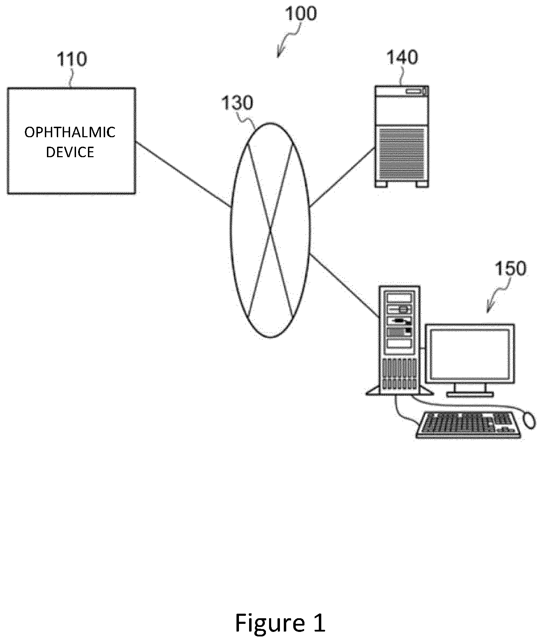

Figure 1 is a diagram illustrating a schematic configuration of an ophthalmic imaging system 100 according to a first exemplary embodiment. As illustrated in Figure 1, the ophthalmic imaging system 100 includes an ophthalmic imaging device 110, a server device (hereafter referred to as "server") 140, and a display device (hereafter referred to as "viewer") 150. In an embodiment the ophthalmic imaging device 110 may be a scanning laser ophthalmoscope (SLO) arranged to capture an ophthalmic image of a patient's eye, such as a fundus image. The server 140 may comprise memory arranged to store ophthalmic images of patient's eyes captured by the ophthalmic imaging device 110. The viewer 150 may be arranged to display the ophthalmic images that are captured by the ophthalmic imaging device 11 and stored on the server 140. The server 140 illustrates an example of an "image processing device" of the present disclosure.

The ophthalmic imaging device 110, the server 140, and the viewer 150 are connected together through a network 130. The network 130 may be a freely selected network such as a LAN, WAN, the internet, a wide area Ethernet, or the like. For example, a LAN may be employed as the network 130 in cases in which the ophthalmic system 100 is built in a single hospital.

The viewer 150 is a client in a client-server system, and one or more such devices may be connected together through the network 130. There may also be one or more servers 140 connected through the network in order to provide system redundancy. Alternatively, if the ophthalmic imaging device 110 is provided with image processing functionality and with the image viewing functionality of the viewer 150, then the ophthalmic images may be acquired and image processing and image viewing may be performed by the ophthalmic imaging device 110 in a standalone state. Moreover, if the server 140 is provided with the image viewing functionality of the viewer 150, then the ophthalmic images may be acquired and image processing and image viewing may be performed by a combination of the ophthalmic imaging device 110 and the server 140.

Note that other ophthalmic equipment (examination equipment for measuring a field of view, measuring intraocular pressure, or the like) and/or a diagnostic support device that analyzes images using artificial intelligence (AI) may be connected to the ophthalmic device 110, the server 140, and the viewer 150 over the network 130.

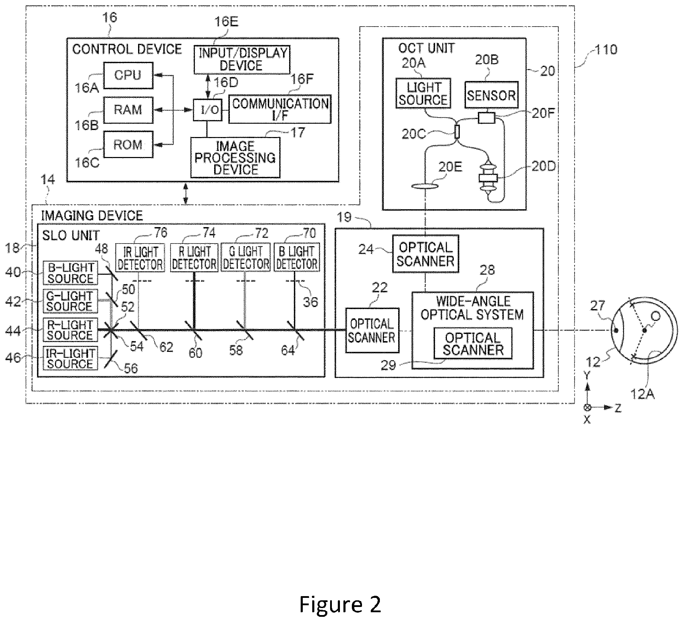

Next, explanation follows regarding an example configuration of the ophthalmic imaging device 110, with reference to Figure 2. For ease of explanation, scanning laser ophthalmoscope is abbreviated to SLO. Moreover, optical coherence tomography is abbreviated to OCT.

With the ophthalmic imaging device 110 installed on a horizontal plane and a horizontal direction taken as an "X direction", a direction perpendicular to the horizontal plane (a vertical direction) is denoted a "Y direction", and a direction connecting the center of the pupil at the anterior eye portion of the examined eye 12 and the center of the eyeball is denoted a "Z direction". The X direction, the Y direction, and the Z direction are mutually orthogonal. The X direction and the Y direction may be determined with reference to the above Z direction.

The ophthalmic imaging device 110 comprises an imaging device 14 and a control device 16 coupled to the imaging device 14. As shown in Figure 2, the imaging device 14 comprises an SLO unit 18 and an OCT unit 20. The SLO unit 18 and OCT unit 20 are arranged to acquire ophthalmic images of the examined eye 12. Two-dimensional fundus images that have been acquired by the SLO unit 18 are referred to below as SLO images. Tomographic images, face-on images (en-face images) and the like of the retina or anterior chamber created based on OCT data acquired by the OCT unit 20 are referred to as OCT images. Note that the imaging device 14 of the ophthalmic imaging device 110 may be a device provided with either the SLO unit 18 or OCT unit 20 alone.

The control device 16 may comprise a computer provided with a Central Processing Unit (CPU) 16A, Random Access Memory (RAM) 16B, Read-Only Memory (ROM) 16C, and an input/output (I/O) port 16D.

The control device 16 may also comprise an input/display device 16E connected to the CPU 16A through the I/O port 16D. The input/display device 16E may include a user interface such as a graphical user interface (not shown) to display images of the examined eye 12 and to receive various inputs from a user, such as a clinician. An example of the graphical user interface is a touch panel display.

The control device 16 may also comprise an image processing device 17 connected to the I/O port 16D. The image processing device 17 is configured to generate ophthalmic images of the examined eye 12 based on data acquired by the imaging device 14. Note that the control device 16 may be connected to the network 130 through a communication interface (I/F) 16F. Note also that the image processing device 17 may include non-illustrated memory. The non-illustrated memory of the image processing device 17 may be employed as memory to store various data under control of the control device 16.

Although, as described above, the control device 16 of the ophthalmic imaging device 110 is provided with the input/display device 16E as illustrated in Figure 2, the embodiments disclosed herein are not limited thereto. For example, a configuration may adopted in which the control device 16 of the ophthalmic imaging device 110 is not provided with the input/display device 16E, and instead a separate input/display device is provided that is physically independent of the ophthalmic imaging device 110. In such cases, the display device is provided with an image processing processor unit that operates under the control of a display control section 204 of the CPU 16A in the control device 16. Such an image processing processor unit may be configured so as to display SLO images and/or OCT image or the like based on image signals output as instructed by the display control section 204.

As shown in Figure 2, the imaging device 14 is coupled to the control device 16 such that the imaging device 14 can operate under the control of the control device 16 and, in particular, under the control of the CPU 16A of the control device 16. The imaging device 14 may include the SLO unit 18 and/or the OCT unit 20, and an imaging optical system 19. The imaging optical system 19 may include one or more of optical scanners 22, 24, and a wide-angle or ultra-wide angle optical system 28. The wide-angle or ultra-wide angle optical system 28 may comprise an optical scanner 29.

The optical scanner 22 is arranged to scan a beam of light emitted from the SLO unit 18 in a specific direction across a region of the patient's eye 12 to image the patient's eye 12. The optical scanner 22 is an optical element capable of scanning or deflecting light beams. The optical scanner 22 may be, for example, any one of: a polygon scanning mirror, a scanning mirror galvanometer, or any other scanning system applicable to ophthalmic imaging. A combination thereof may also be employed. The optical scanner 29 may scan incident light in a direction intersecting with the specific direction. As long as the optical scanner 29 is an optical element capable of deflecting light beams, it may be configured by any out of, for example, a polygon mirror, a mirror galvanometer, or the like. A combination thereof may also be employed. The optical scanners 22, 29 accordingly perform two-dimensional scanning of light emitted from the SLO unit 18, in the X direction and the Y direction.

The optical scanner 24 scans light emitted from the OCT unit 20 in a specific direction across a region of the patient's eye 12 to image the patient's eye 12. The optical scanner 24 is an optical element capable of scanning or deflecting light beams. The optical scanner 24 may be, for example, any one of: a polygon scanning mirror, a scanning mirror galvanometer, or any other scanning system applicable to ophthalmic imaging. A combination thereof may also be employed. The optical scanners 24, 29 accordingly scan light emitted from the OCT unit 20 two dimensionally, in the X direction and the Y direction.

Note that the optical scanners 22, 24 are not limited to being configured by different optical scanner, and a common optical scanner may be employed.

Moreover, the wide-angle or ultra-wide angle optical system 28 guides light from the SLO unit 18 and light from the OCT unit 20 to the examined eye. Detailed explanation follows regarding the imaging optical system 19 including the wide-angle optical system 28.

Note that the wide-angle optical system 28 may be a reflection optical system employing one or more concave mirrors such as a second reflection mirror, a refraction optical system employing a wide-angle lens, or may be a reflection-refraction optical system employing a combination of a concave mirror and a lens. Employing a wide-angle optical system that utilizes a second reflection mirror, wide-angle lens, or the like enables imaging to be performed not only of a central portion of the fundus, but also of the retina at the fundus periphery.

For a system including a second reflection mirror, a configuration may be adopted that utilizes a second reflection mirror system as disclosed in International Publication (WO) Nos. 2016/103484 or 2016/103489. The disclosures of WO Nos. 2016/103484 and 2016/103489 are incorporated in their entirety in the present specific by reference herein.

Observation of the fundus over a wide field of view (FOV) 12A may be implemented by the wide-angle optical system 28. The FOV 12A refers to a range capable of being imaged by the imaging device 14. The FOV 12A may be expressed as a viewing angle. In the present exemplary embodiment the viewing angle may be defined in terms of an internal illumination angle and an external illumination angle. The external illumination angle is the angle of illumination by a light beam illuminated from the ophthalmic device 110 toward the examined eye 12, and is an angle of illumination defined with respect to a pupil 27. The internal illumination angle is the angle of illumination of a light beam illuminated onto the fundus, and is an angle of illumination defined with respect to an eyeball center O. Correspondence relationships exist between the external illumination angle and the internal illumination angle. For example, an external illumination angle of 120° corresponds to an internal illumination angle of about 160°. The internal illumination angle in the present exemplary embodiment is 200°.

SLO fundus images obtained by imaging at an imaging angle of view having an internal illumination angle of 160° or greater are referred to as UWF-SLO fundus images. UWF is an abbreviation for ultra-wide field (ultra-wide angled). A region extending from a posterior pole portion of a fundus of the examined eye 12 past an equatorial portion thereof can be imaged by the wide-angle optical system 28 having a field of view (FOV) angle of the fundus that is an ultra-wide field, enabling imaging of structural objects, such as vortex veins, present at fundus peripheral portions.

The ophthalmic imaging device 110 may be capable of imaging a region 12A with an internal illumination angle of up to approximately 200° with respect to the eyeball center O of the examined eye 12 as a reference position. Note that an internal illumination angle of 200° corresponds to an external illumination angle of 110° with respect to the pupil of the eyeball of the examined eye 12 as the reference. Namely, the wide-angle optical system 28 illuminates laser light through the pupil at an angle of view of an external illumination angle of 110° in order to image a fundus region with an internal illumination angle of 200°.

An example SLO system may comprise the control device 16, the SLO unit 18, and the imaging optical system 19 as illustrated in Figure 2. The SLO system is provided with the wide-angle optical system 28, enabling fundus imaging over the wide FOV 12A. The skilled reader would understand that in an embodiment that did not comprise the OCT unit 20 then optical scanner 24 may be omitted. Similarly, in an embodiment that did not comprise the SLO unit 18 then optical scanner 22 may be omitted.

The SLO unit 18 may be provided with one or more of a blue (B) light source 40, a green (G) light source 42, a red (R) light source 44, an infrared (for example near infrared) (IR) light source 46, and optical systems 48, 50, 52, 54, 56 to guide the light from the light sources 40, 42, 44, 46 onto a single light path using reflection and/or transmission. In the present example embodiment, the optical systems 48, 56 are mirrors, and the optical systems 50, 52, 54 are beam splitters. B light is reflected by the optical system 48, is transmitted through the optical system 50, and is reflected by the optical system 54. G light is reflected by the optical systems 50, 54, R light is transmitted through the optical systems 52, 54, and IR light is reflected by the optical systems 52, 56. The respective lights are thereby guided onto a single light path.

The SLO unit 18 is configured so as to be capable of switching between light sources for emitting laser light of different wavelengths or a combination of light-emitting light sources, such as a mode in which R light and G light are emitted, a mode in which infrared light is emitted, etc. Although the example in Figure 2 includes four light sources, i.e. the B light source 40, the G light source 42, the R light source 44, and the IR light source 46, the present disclosure is not limited thereto. For example, the SLO unit 18 may further include a white light source, in a configuration in which light is emitted in various modes, such as a mode in which G light, R light, and B light are emitted, and a mode in which white light is emitted alone.

Light incident to the imaging optical system 19 from the SLO unit 18 is scanned in the X direction and the Y direction by the optical scanner 22. The scanning light passes through the wide-angle optical system 28 and the pupil 27 and is illuminated onto the fundus. Reflected light that has been reflected by the fundus passes through the wide-angle optical system 28 and the optical scanner 22 and is incident to the SLO unit 18.

The SLO unit 18 is provided with a beam splitter 64 and a beam splitter 58. From out of the light coming from a posterior eye portion (fundus) of the examined eye 12, the B light therein is reflected by the beam splitter 64 and light other than B light therein is transmitted by the beam splitter 64. From out of the light transmitted by the beam splitter 64, the G light therein is reflected by the beam splitter 58 and light other than G light therein is transmitted by the beam splitter 58. The SLO unit 18 is further provided with a beam splitter 60 that, from out of the light transmitted through the beam splitter 58, reflects R light therein and transmits light other than R light therein. The SLO unit 18 is further provided with a beam splitter 62 that reflects IR light from out of the light transmitted through the beam splitter 60. The SLO unit 18 is further provided with a B light detector 70 to detect B light reflected by the beam splitter 64, a G light detector 72 to detect G light reflected by the beam splitter 58, an R light detector 74 to detect R light reflected by the beam splitter 60, and an IR light detector 76 to detect IR light reflected by the beam splitter 62.

Note that in order to separate illuminated light from a light source and reflected light from the examined eye, a reflection element may be applied for the beam splitter 58, 60, 62, 64 such as a hole mirror, a dot mirror, or the like provided with a hole at a part including a location where light of a specific wavelength is transmitted, with other locations thereof being locations where such light is reflected. For example, a hole mirror that transmits light from the B light source 40 and reflects reflected light from the examined eye may be applied as the beam splitter 64. The hole mirror employed in the beam splitter 64 may have a configuration in which a hole portion is arranged on the optical axis of B light so as to transmit light from the B light source 40.

Light that has passed through the wide-angle optical system 28 and the optical scanner 22 and been incident to the SLO unit 18 (i.e. reflected light that has been reflected by the fundus) is reflected by the beam splitter 64 and photo-detected by the B light detector 70 when B light, and is transmitted through the beam splitter 64, reflected by the beam splitter 58, and photo-detected by the G light detector 72 when G light. The incident light when R light is transmitted through the beam splitters 64, 58, reflected by the beam splitter 60, and photo-detected by the R light detector 74. The incident light when IR light is transmitted through the beam splitters 64, 58, 60, reflected by the beam splitter 62, and photo-detected by the IR light detector 76. The image processing device 17 that operates under the control of the CPU 16A employs signals detected by the B light detector 70, the G light detector 72, the R light detector 74, and the IR light detector 76 to generate UWF-SLO images.

UWF-SLO images generated using signals detected by the B light detector 70 are called B-UWF-SLO images (blue fundus images). UWF-SLO images generated using signals detected by the G light detector 72 are called G-UWF-SLO images (green fundus images). UWF-SLO images generated using signals detected by the R light detector 74 are called R-UWF-SLO images (red fundus images). UWF-SLO images generated using signals detected by the IR light detector 76 are called IR-UWF-SLO images (IR fundus images). UWF-SLO images encompass the red fundus images, the green fundus images, the blue fundus images, and the IR fundus images. Florescent light UWF-SLO images imaged with florescent light are also encompassed therein.

The control device 16 may also be arranged to control the light sources 40, 42, 44 such that one or more of the light sources 40, 42, 44 emit light simultaneously. A green fundus image, a red fundus image, and a blue fundus image are obtained with mutually corresponding positions by imaging the fundus of the examined eye 12 at the same time with the B light, G light, and R light. An RGB color fundus image is obtained from the green fundus image, the red fundus image, and the blue fundus image. The control device 16 obtains a green fundus image and a red fundus image with mutually corresponding positions by controlling the light sources 42, 44 so as to emit light at the same time and by imaging the fundus of the examined eye 12 at the same time with the G light and R light. An RG color fundus image is obtained from the green fundus image and the red fundus image. Moreover, a full color fundus image may be generated using the green fundus image, the red fundus image, and the blue fundus image.

A region extending from a posterior pole portion of a fundus of the examined eye 12 past an equatorial portion thereof can be imaged by the wide-angle optical system 28 with a field of view (FOV) angle of the fundus that is an ultra-wide field.

An OCT system may be implemented by the control device 16, the OCT unit 20, and the imaging optical system 19 illustrated in Figure 2. The OCT system includes the wide-angle optical system 28, and is accordingly able to perform OCT imaging of fundus peripheral portions similarly to the imaging of SLO fundus images described above. Namely, OCT imaging over a region extending from a posterior pole portion of the examined eye 12 fundus past the equatorial portion 178 is able to be performed by employing the wide-angle optical system 28 having a field of view (FOV) angle of the fundus that is an ultra-wide field. OCT data of structural objects such as vortex veins present in the fundus peripheral portions can be acquired, and tomographic images of vortex veins and a 3D structure of vortex veins can be obtained by performing image processing on the OCT data.

The OCT unit 20 shown in Figure 2 comprises a light source 20A, a sensor (detection element) 20B, a first light coupler 20C, a reference optical system 20D, a collimator lens 20E, and a second light coupler 20F.

Light emitted from the light source 20A is split by the first light coupler 20C. One part of the split light is collimated by the collimator lens 20E into parallel light serving as measurement light before being incident to the imaging optical system 19. The measurement light is illuminated onto the fundus through the wide-angle optical system 28 and the pupil 27. Measurement light that has been reflected by the fundus passes through the wide-angle optical system 28 so as to be incident to the OCT unit 20, then passes through the collimator lens 20E and the first light coupler 20C before being incident to the second light coupler 20F.

The other part of the light emitted from the light source 20A and split by the first light coupler 20C is incident to the reference optical system 20D as reference light, and is made incident to the second light coupler 20F through the reference optical system 20D.

The respective lights that are incident to the second light coupler 20F, namely the measurement light reflected by the fundus and the reference light, interfere with each other in the second light coupler 20F so as to generate interference light. The interference light is photo-detected by the sensor 20B. The image processing device 17 operating under the control of an image processing section 206 generates OCT data detected by the sensor 20B. OCT images, such as tomographic images and en-face images, are generated in the image processing device 17 based on this OCT data.

The OCT unit 20 is able to scan a specific range (for example a rectangular range of 6 mm × 6 mm) at a single time of OCT imaging. The specific range is not limited to being 6 mm × 6 mm, and may be a square range of 12 mm × 12 mm or 23 mm × 23 mm, may be a rectangular range of 14 mm × 9 mm, 6 mm × 3.5 mm, or the like, and may be a freely selected rectangular range. Moreover, the specific range may be a circular range having a diameter of 6 mm, 12 mm, 23 mm, or the like.

By employing the wide-angle optical system 28, the ophthalmic device 110 is able to designate the region 12A having an internal illumination angle of 200° as the scan target. Namely, OCT imaging is performed of the specific range including vortex veins, for example, by controlling the optical scanners 24, 29. The ophthalmic device 110 is able to generate OCT data by this OCT imaging.

Thus, the ophthalmic device 110 is able to generate OCT images such as tomographic images of the fundus including vortex veins (B-scan images), OCT volume data including vortex veins, and en-face images that are cross-sections of such OCT volume data (face-on images generated based on the OCT volume data). It will be understood that the term "OCT images" also encompasses an OCT image of a fundus center portion (posterior pole portion of the eyeball where the macular, the optic nerve head, and the like are present).

The OCT data (or image data of the OCT images) may be sent from the ophthalmic device 110 to the server 140 though the communication interface 16F and is stored in a storage device 254. Note that although in the present exemplary embodiment an example is given in which the light source 20A is a wavelength swept-source OCT (SS-OCT), various types of OCT system may be employed, such as a spectral-domain OCT (SD-OCT) or a time-domain OCT (TD-OCT) system.

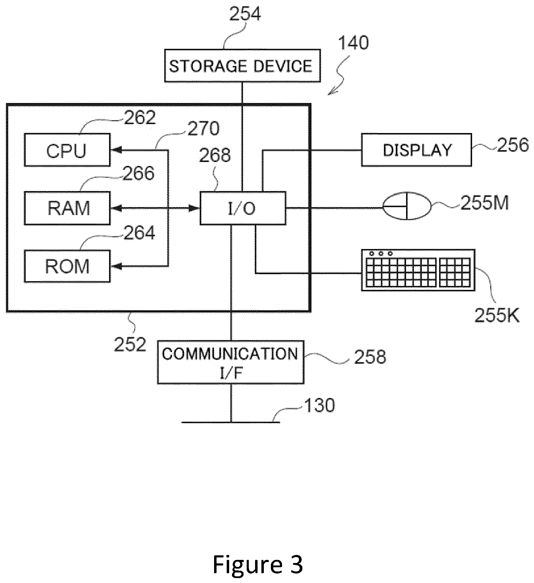

Next, description follows regarding a configuration of an electrical system of the server 140, with reference to Figure 3. As illustrated in Figure 3, the server 140 includes a computer main body 252. The computer main body 252 includes a CPU 262, RAM 266, ROM 264, and an input/output (I/O) port 268. The CPU 262, the RAM 266, the ROM 264, and the input/output port (I/O) 268 may be connected so as to be capable of communicating with each other through a bus 270. The storage device 254, a display 256, a mouse 255M, a keyboard 255K, and a communication interface (I/F) 258 are connected to the input/output (I/O) port 268. The storage device 254 is, for example, configured by non-volatile memory. The input/output (I/O) port 268 is connected to the network 130 through the communication interface (I/F) 258. The server 140 is accordingly able to communicate with the ophthalmic device 110 and the viewer 150.

An image processing program is stored in the ROM 264 or the storage device 254. The ROM 264 and the storage device 254 are examples of "memory" of the present disclosure. The CPU 262 is an example of a "processor" of the present disclosure. The image processing program is an example of a "program" of the present disclosure.

The server 140 stores respective data received from the ophthalmic device 110 in the storage device 254. Moreover, the server 140 is able to use the various data received to store data of a process related to image processing and processing result data in the storage device 254.

Description follows regarding various example functions implemented by the CPU 262 of the server 140 executing the image processing program.

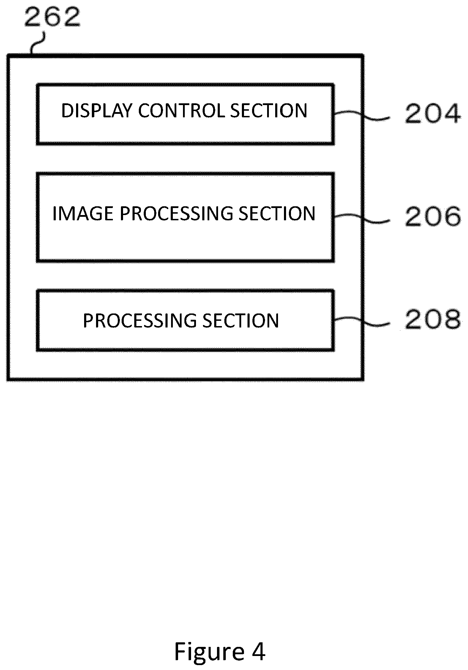

The image processing program executed in the CPU 262 may include respective sections to implement one or more of: a display control function, an image processing function, and a processing function. Specifically, by the CPU 262 executing the image processing program, the CPU 262 operates, as illustrated in Figure 4, as the display control section 204 to implement a display control function, the image processing section 206 to implement an image processing function, and a processing section 208 to implement a processing function.

Next, description follows regarding image processing by the server 140. The CPU 262 of the server 140 implements image processing (an image processing method) by executing the image processing program.

First, by executing the image processing program, the CPU 262 performs processing in the image processing section 206 to acquire a fundus image from the storage device 254. This fundus image includes, for example, data related to blood vessels, such as choroidal vessels including vortex veins. Next, the CPU 262 performs processing in the image processing section 206 to execute various predetermined image processing on the acquired fundus image, such as emphasizing blood vessels in the fundus image, generating a three-dimensional image of choroidal vessels, and the like. Next, the CPU 262 performs processing in the processing section 208 to output an image generated after the image processing, specifically processing to save the image in the RAM 266 or the storage device 254, and then ends the image processing.

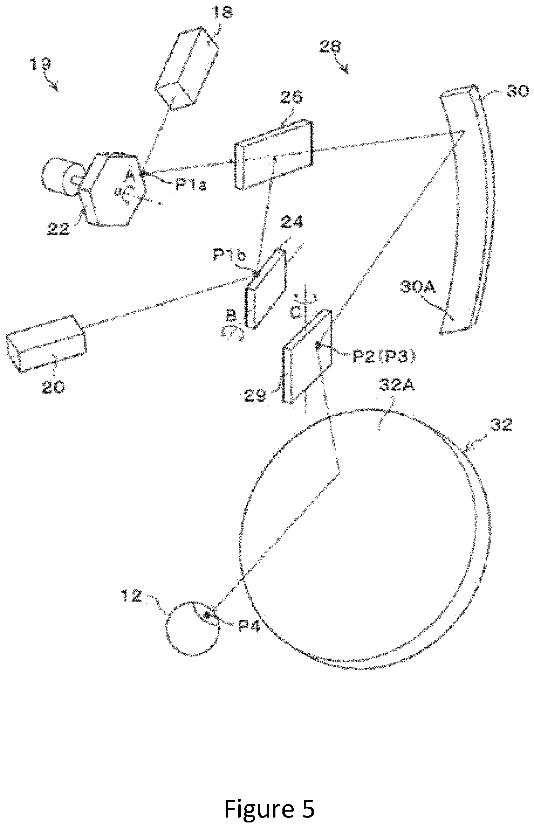

Explanation follows regarding the example embodiment of the imaging optical system 19 included in the wide-angle optical system 28, with reference to Figure 5. The wide-angle optical system 28 may be configured to include a first reflection mirror 30 and a second reflection mirror 32, as illustrated in Figure 5. The first and second reflection mirrors 30, 32 may either or both be elliptical mirrors.

As shown in Figure 5, the optical scanner 22 may be, for example, a polygon scanning mirror arranged to scan light emitted from the SLO unit 18 in the Y direction by rotating in the direction of arrow A. The optical scanner 24 may be, for example, a scanning mirror galvanometer arranged to scan light from the OCT unit 20 in the Y direction by rotating in the direction of arrow B.

The wide-angle optical system 28 may include a dichroic mirror 26, a first reflection mirror 30 having an elliptical concave reflection face, the optical scanner 29, for example a scanning mirror galvanometer, and the second reflection mirror 32 having an elliptical concave reflection face.

The dichroic mirror 26 is arranged to transmit light emitted from the SLO unit 18 and to reflect light emitted from the OCT unit 20, thereby guiding both light beams onto a common light path that can propagate through the wide-angle optical system 28 to the patient's eye 12. As shown in Figure 5, light emitted from the SLO unit 18 is aligned with the common light path such that when the dichroic mirror 26 transmits the light emitted from the SLO unit 18 that light can travel along the common light path. The dichroic mirror 26 is orientated such that light emitted by the OCT unit 20 is reflected by the dichroic mirror 26 along the common light path towards the patient's eye 12. The skilled person will understand that the dichroic mirror 26 is an optional feature and, for example, in an ophthalmic imaging system with only SLO imaging functionality the dichroic mirror 26 may be omitted.

The dichroic mirror 26 is arranged to transmit light emitted from the SLO unit 18 and to reflect light emitted from the OCT unit 20, thereby guiding both light beams onto a common light path that can propagate through the wide-angle optical system 28 to the patient's eye 12. As shown in Figure 5, light emitted from the SLO unit 18 is aligned with the common light path such that when the dichroic mirror 26 transmits the light emitted from the SLO unit 18 that light can travel along the common light path. The dichroic mirror 26 is orientated such that light emitted by the OCT unit 20 is reflected by the dichroic mirror 26 along the common light path towards the patient's eye 12. The skilled person will understand that the dichroic mirror 26 is an optional feature and, for example, in an ophthalmic imaging system with only SLO imaging functionality the dichroic mirror 26 may be omitted.

The first elliptical mirror 30 reflects the light incident to a mirror face 30A, and thereby guides this reflected light to the optical scanner 29. The optical scanner 29 scans incident light in the X direction by rotational oscillation in the arrow C direction.

The first reflection mirror 30 and the second reflection mirror 32 are examples of an "elliptical mirror" of the present disclosure. Each of the elliptical mirrors comprise two focal points. The optical scanner 22 is located at a first focal point of the first mirror 30 and the optical scanner 29 is located at a second focal point of the first mirror 30. The second mirror is positioned such that the optical scanner 29 is also located at a first focal point of the second mirror 32 and a patient's pupil 27 is located at the second focal point of the second mirror 32 when imaging the patient's eye 12.

The second reflection mirror 32 reflects incident light by a mirror face 32A so as to guide this reflected light onto the fundus of the examined eye 12. Light incident on the fundus (retina) is reflected by the fundus and the reflected light propagates through the wide-angle optical system 28 via the common light path, to the detectors located in the SLO unit 18 and OCT unit 20 such that an image of the fundus can be generated. The reflected light is guided through the wide-angle optical system 28 to the dichroic mirror 26 along the same light path to that of the light described above, but in the opposite direction. The dichroic mirror 26 guides the fundus reflected light arising from the light from the SLO unit 18 into the SLO unit 18, and guides the fundus reflected light arising from the light from the OCT unit 20 into the OCT unit 20.

The first elliptical mirror 30 and the second elliptical mirror 32 are examples of a "reflection mirror" of the present disclosure.

In the wide-angle optical system 28 described above, the optical scanner 22 and the optical scanner 24 are arranged such that a scanning central position P1a of light from the SLO unit 18 in the optical scanner 22 is conjugate to a scanning central position P1b of light in the optical scanner 24. Moreover, in the wide-angle optical system 28, the optical scanner 22, the optical scanner 24, and the optical scanner 29 are arranged such that a scanning central position P2 of light in the optical scanner 29 is conjugate to the scanning central positions P1a, P1b of light of the optical scanner 22 and the optical scanner 24. The pupil of the eyeball of the examined eye 12 is positioned at one of the focal positions of the second elliptical mirror 32.

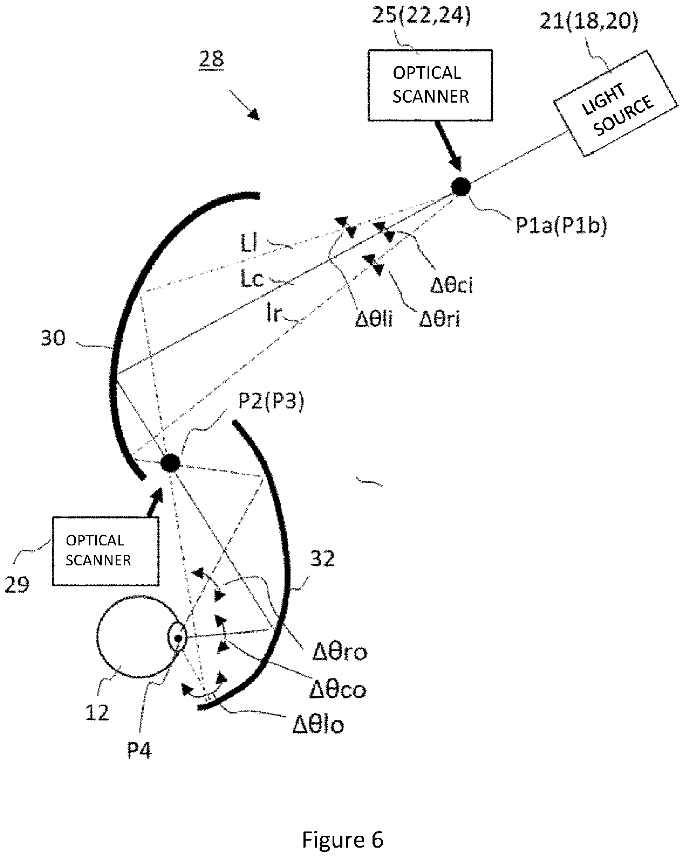

The optical scanner 29 is arranged such that a scanning central position P2 of light in the optical scanner 29 is at a pupil conjugate position P3 conjugate to a pupil position P4 (pupil conjugate position) of the examined eye 12 formed by the second elliptical mirror 32, this being the other focal position of the second elliptical mirror 32. The first elliptical mirror 30 and the optical scanner 29 are arranged such that one focal point of the first elliptical mirror 30 with the elliptical concave reflection face is positioned at the scanning central position P2 of light in the optical scanner 29. The pupil conjugate position P3 due to the elliptical mirror 32, and the pupil conjugate position P2 due to the first elliptical mirror 30, are accordingly arranged so as to be a common position (see Figure 6). The optical scanners 22, 24 are arranged such that the scanning central positions P1a, P1b of light in the optical scanners 22, 24 are at a pupil conjugate position P1 conjugate to the pupil conjugate position P2 formed by the first elliptical mirror 30, this being the other focal position of the first elliptical mirror 30.

However, when scanning a fundus or the like of the examined eye, for example, the light from light sources of the SLO unit 18 and the OCT unit 20 should be incident to the examined eye as a parallel light beam. However, in cases in which the wide-angle optical system 28 is configured including elliptical mirrors, a ratio (Δθro/Δθri) of a small change (Δθro) of incident angle of incident light at a position of the pupil of the examined eye with respect to a small change (Δθri) of incident angle in the vicinity of an end portion of the scan range is greater than a ratio (Δθco/Δθci) of a small change (Δθco) of incident angle of incident light at a position of the pupil of the examined eye with respect to a small change (Δθci) of incident angle in the vicinity of the center of the scan range. In other words, a small change in scan angle when the scanner is orientated in a central region of the field of view leads to a relatively small change in scan angle at the eye compared to a similarly small change in scan angle when the scanner is orientated in a peripheral region of the field of view which results in a relatively large change in scan angle at the eye. This leads to issue such as distortion arising in captured images, a darker image at the image periphery than at the center portion thereof, and the like.

This will now be explained more specifically, with reference to Figure 6. Consider light Lc in the vicinity of a scanning center of scanning (vertical scanning) in an optical scanner 25 (22, 24), light Lr at a vicinity of an end portion in a first direction looking from the center along the scan progression direction, and light LI at a vicinity of an end portion in a second direction, which is the opposite direction to the first direction. The light Lc, the light Lr, and the light LI are all reflected at the first elliptical mirror 30, further reflected by the second elliptical mirror 32, and arrive at a position of the pupil of the examined eye 12.

When this occurs, for the light Lc, when the scanning direction by the optical scanner 25 has changed by a small angle Δθci, then the scanning direction at the vicinity of the pupil of the examined eye 12 changes by a small angle Δθco (a ratio of change of Δθco/ Δθci). However, for the light Lr, when the scanning direction by the optical scanner 25 changes by a small angle Δθri, then the scanning direction at the vicinity of the examined eye 12 changes by a small angle Δθro (a ratio of change of Δθro/ Δθri). Moreover, for the light Ll, when the scanning direction by the optical scanner 25 has changed by a small angle Δθli, then the scanning direction at the vicinity of the examined eye 12 changes by a small angle Δθlo (a ratio of change of Δθlo/ Δθli).

Regarding these ratios of change, Δθro/ Δθri and Δθlo/ Δθli are greater than Δθco/ Δθci. Given such a relationship between ratios of change, captured images obtained by the SLO unit 18 and/or the OCT unit 20 have a large amount of distortion at peripheral portions thereof, and are also images with differences in light exposure amount (angular distortion).

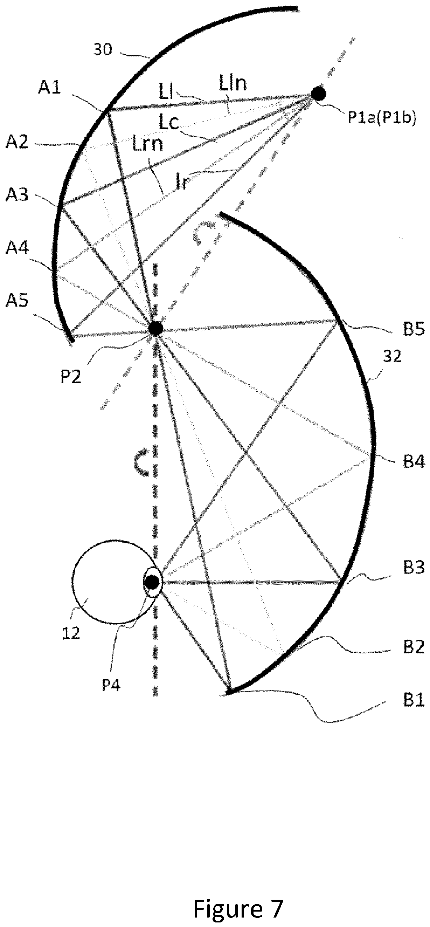

Thus in the present exemplary embodiment, the first elliptical mirror 30 and the second elliptical mirror 32 are designed so as to achieve the following relationships such that such distortion and difference in light exposure amount do not occur. This will now be explained with reference to Figure 7.

First, for the light Lr, distances between points are defined as set out below:
d1: a distance from the scanning center position P1a (first condensing spot) to a reflection point A5 on the first elliptical mirror 30.
d2: a distance from the reflection point A5 to the scanning center position P2 (second condensing spot).
d3: a distance from the scanning center position P2 to a reflection point B5 of the second elliptical mirror 32.
d4: a distance from reflection point B5 to the pupil position P4 (third condensing spot).

Moreover, for the light Ll, distances between points are defined as set out below:
d1': a distance from the scanning center position P1a (first condensing spot) to a reflection point A1 on the first elliptical mirror 30.
d2': a distance from the reflection point A1 to the scanning center position P2 (second condensing spot).
d3': a distance from the scanning center position P2 to a reflection point B1 of the second elliptical mirror 32.
d4': a distance from reflection point B1 to the pupil position P4 (third condensing spot).

In this case, the first elliptical mirror 30 and the second elliptical mirror 32 may be designed such that d1, d2, d3, and d4 are related by d2 being shorter than d1 and d3 being shorter than d4 (a long-short/short-long relationship), and such that d1', d2', d3', and d4', are related by d1' being shorter than d2' and d4' being shorter than d3' (a short-long/long-short relationship). Design of the first elliptical mirror 30 and the second elliptical mirror 32 in this case includes the positional relationship (angle, separation distance, and the like) between each other, major axis, minor axis, first focal length/second focal length, and the like.

Alternatively, as an opposite situation thereto, the first elliptical mirror 30 and the second elliptical mirror 32 may be set such that d1, d2, d3, and d4 are related by d2 being longer than d1 and d3 being longer than d4 (a short-long/long-short relationship), and such that d1', d2', d3', and d4' are related by d1' being longer than d2' and d4' being longer than d3' (a long-short/short-long relationship).

This thereby enables captured image distortion and the like arising from angular distortion to be suppressed due to the light Lr and the light LI having relationships to each other that are respectively a long-short/short-long or a short-long/long-short relationship.

As shown in Figure 7, the first elliptical mirror 30 and second elliptical mirror 32 are orientated such that the concave side of each elliptical mirror 30, 32 face each other. As the light beam is scanned by the scanner via P1 angular distortion imparted on the light beam by the first elliptical mirror 30 is substantially cancelled out by the equal and opposite angular distortion imparted on the light beam by the second elliptical mirror 32. The opposing first and second elliptical mirrors 30, 32 beneficially impart substantially symmetric angular distortion on the imaging light beam which in turn has the effect of cancelling or suppressing the effects of angular distortion.

Note that although the above description has focused on the light Lr, LI at the end portions of a scan range (V scan), the same explanation to above also applies to relationships between the light Lc and the light Lr, or to relationships between light Lrn and light Lln that are intermediate between the light Lc and the light Lr or the light LI (Figure 7).

### Second Exemplary Embodiment

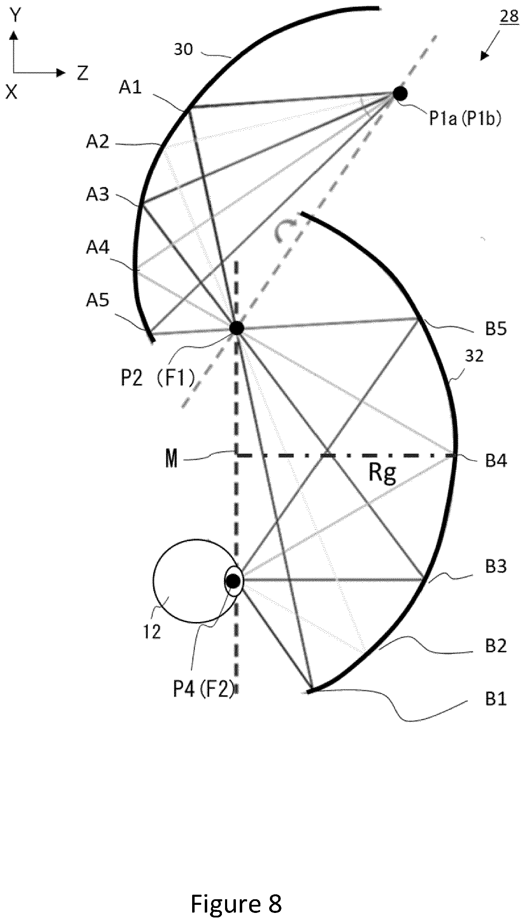

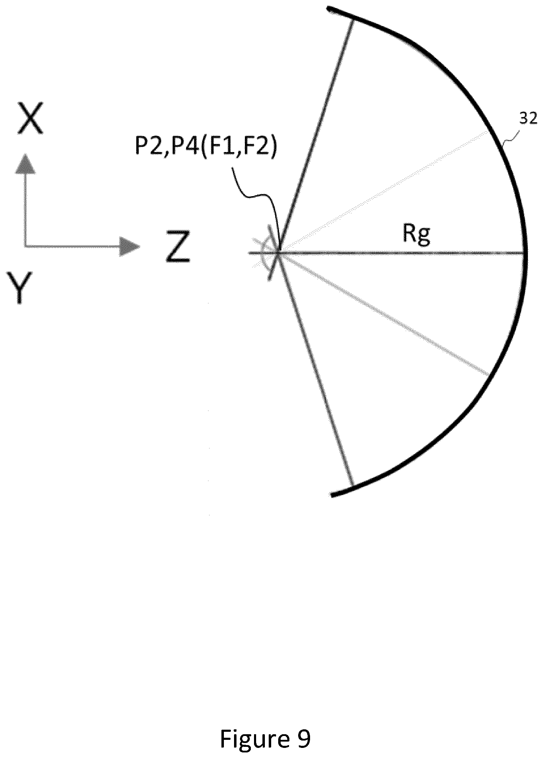

Next, description follows regarding an ophthalmic system 100 according to a second exemplary embodiment, with reference to Figure 8 and Figure 9. An outline configuration of the ophthalmic system 100 and an outline configuration of the wide-angle optical system 28 (Figure 1 to Figure 5) are the same as in the first exemplary embodiment, and duplicate explanation thereof will be omitted. The ophthalmic system 100 of the second exemplary embodiment differs from the first exemplary embodiment in elliptical mirror configuration in the wide-angle optical system 28.

Figure 8 is a schematic side view taken along a YZ plane of the wide-angle optical system 28, and Figure 9 is a plan view taken along an XZ plane of the second elliptical mirror 32 of the wide-angle optical system 28. The second elliptical mirror 32 is an elliptical mirror with a first focal point F1 and a second focal point F2, and while having an elliptical cross-section in the YZ plane, it has a true circle cross-section profile (radius Rxz) in the XZ plane, as illustrated in Figure 9. Moreover, the length of a line RG extending between an apex of the reflection face of the second elliptical mirror 32 from and a line connecting the first focal point F1 and the second focal point F2 is equivalent to the true circle radius Rxz (Rxz = RG) where the line RG is perpendicular to the line connecting the first and second focal points F1, F2. The diameter of the circle in the horizontal direction (XZ plane) of the second elliptical mirror 32 may be equal to the minor axis of the ellipse of the second elliptical mirror 32.

By adopting such a configuration, as illustrated in Figure 9, incident light converged at the first focal point F1 is, in the XZ plane (H scan direction), reflected straight back in the same direction and converged at the second focal point F2, due to being incident perpendicular to the cross-section of the second elliptical mirror 32 having the true circle profile in the XZ plane across the entire scan range. Due to the reflection conditions being the same in all scanning directions, distortion or the like does not arise in the captured images, enabling excellent images of the examined eye to be obtained across a wide-angle range.

The present invention is not limited by the above examples, and encompasses various modified examples. For example, although the above examples have been described in detail to facilitate understanding of the present invention, there is no limitation to always including all of the configuration described. Moreover, parts of configuration of one example may be replaces with configuration of another example, moreover configuration of one example may be added to configuration of another example.

## Claims

1. An ophthalmic imaging device (110) comprising:
an imaging device (14) that is arranged to project light from a light source (20A, 40, 42, 44, 46) onto an examined eye (12), and that is further arranged to detect light from the examined eye (12) to acquire an ophthalmic image of the examined eye (12), wherein:
the imaging device (14) comprises:
a scanner (22, 24) that is arranged to scan light from the light source, and
a wide-angle optical system (28) that projects light scanned by the scanner (22, 24) onto a retina of the examined eye (12) at a wide angle; wherein:
the wide-angle optical system (28) comprises:
a first elliptical mirror (30) arranged to reflect light scanned by the scanner (22, 24), and
a second elliptical mirror (32) arranged to reflect light from the first elliptical mirror (30) so as to reflect light toward the examined eye (12); wherein:
for a first light (Ll)that is on a first side looking from center light (Lc) of a scanning center by the scanner (22, 24), d1 is a distance from a first condensing spot (P1a) to a first reflection point (A1) on the first elliptical mirror (30), d2 is a distance from the first reflection point (A1) to a second condensing spot (P2), d3 is a distance from the second condensing spot (P2) to a second reflection point (B1) of the second elliptical mirror (32), and d4 is a distance from the second reflection point (B1) to a third condensing spot (P4);
for a second light (Lr) that is on a second side, which is the opposite side to the first side, looking from the center light (Lc) of the scanning center by the scanner (22, 24), d1' is a distance from a first condensing spot (P1a) to a first reflection point (A5) on the first elliptical mirror (30), d2' is a distance from the first reflection point (A5) to a second condensing spot (P2), d3' is a distance from the second condensing spot (P2) to a second reflection point (B5) of the second elliptical mirror (32), and d4' is a distance from the second reflection point (B5) to a third condensing spot (P4); and
the first elliptical mirror (30) and the second elliptical mirror (32) are set such that either of the following sets of relationships holds:
d1, d2, d3, and d4 are related by d2 being shorter than d1 and d3 being shorter than d4, and d1', d2', d3', and d4' are related by d1' being shorter than d2' and d4' being shorter than d3'; or
d1, d2, d3, and d4 are related by d2 being longer than d1 and d3 being longer than d4, and d1', d2', d3', and d4' are related by d1' being longer than d2' and d4' being longer than d3'.

2. An ophthalmic imaging device (110) comprising:
an imaging device (14) that is arranged to project light from a light source (20A, 40, 42, 44, 46) onto an examined eye (12), and that is further arranged to detect light from the examined eye (12) to acquire an ophthalmic image of the examined eye (12), wherein:
the imaging device (14) comprises:
a scanner (22, 24) arranged to scan light from the light source (20A, 40, 42, 44, 46), and
a wide-angle optical system (28) that projects light scanned by the scanner (22, 24) onto a retina of the examined eye (12) at a wide angle;
the wide-angle optical system (28) comprising:
a first elliptical mirror (30) arranged to reflect light scanned by the scanner (22, 24), and
a second elliptical mirror(32) arranged to reflect light from the first elliptical mirror (30) so as to reflect light toward the examined eye (12), wherein:
the second elliptical mirror (32) comprises an elliptical profile in a first plane extending through a first focal point (P2) and a second focal point (P4), and a circular profile in a second plane that is orthogonal to the first plane; and
a first line extending between the first focal point (P2) and the second focal point (P4) is a substantially equal length to a second line (Rg) extending perpendicularly from the first line to an apex of the second elliptical mirror.
